# EUROPEAN PATENT APPLICATION

(11) **EP 4 257 077 A1**
(43) Date of publication of application: **11.10.2023**
(21) Application number: 21903416.2
(22) Date of filing: 07.12.2021
(51) Int. Cl.: A61C 8/00

(54) **ANALOG HOLDING JIG**

(30) Priority: 07.12.2020 JP 2020203006
(71) Applicant: E-Joint Corporation, Iruma-shi, Saitama 358-0011 (JP)
(72) Inventor: FUJIKAWA Satoru, Iruma-shi, Saitama 358-0011 (JP); NATSUBORI Reiji, Hachinohe-shi, Aomori 031-0072 (JP)
(74) Representative: Peters, Sebastian Martinus
(86) International application number: PCT/JP2021/045011
(87) International publication number: WO 2022/124317

(57) **Abstract**

[Problem] To provide a jig or the like, which makes it easier to install a dental analog in a jaw part model. [Solution] An analog holding jig 13 which comprises: a holding part main body 5 for holding a dental analog 3; and an analog guide connection part 9 which is connected to the holding part main body 5 for the connection of an analog guide 7. This analog holding jig 13 is used for the purpose of planting the dental analog 3 in a jaw part model 11 by the intermediary of the analog guide 7.

## Description

### TECHNICAL FIELD

This invention relates to a dental jig or the like.

### BACKGROUND ART

Japanese Patent No. 6208905 discloses a scanning jig. It is known that a scanning jig is thus used when an implant is manufactured. For example, when an implant is manufactured as disclosed in Japanese Patent No. 6161048, a dental analog (implant analog) is used. There has been a problem that, when the dental analog is installed to a jaw part model, it is difficult to adjust an angle or a position of the dental analog.
Patent Document 1: Japanese Patent No. 6208905
Patent Document 2: Japanese Patent No. 6161048

### DISCLOSURE OF THE INVENTION

### PROBLEMS TO BE SOLVED BY THE INVENTION

Therefore, a jig or the like that facilitates installing the dental analog to the jaw part model has been desired.

### SOLUTIONS TO THE PROBLEMS

The first invention relates to an analog holding jig.

The analog holding jig is a device used for holding a dental analog 3 and implanting the dental analog 3 into a jaw part model 11 via an analog guide 7.

An analog holding jig 13 includes a holding portion main body 5 for holding the dental analog 3 and an analog guide connecting portion 9 coupled to the holding portion main body 5 for connecting to the analog guide 7.

The second invention relates to an analog guide.

The analog guide is a device used for implanting the dental analog 3 into the jaw part model 11.

The analog guide 7 includes an analog guide main body 15 and an analog holding jig connecting portion 17.

The analog guide main body 15 is a portion having a shape to be installable to the jaw part model 11.

The analog holding jig connecting portion 17 is a portion disposed in the analog guide main body 15, connected to a connecting portion for the analog guide 7 of the analog holding jig 13 to cause the dental analog 3 held to the analog holding jig 13 to face an analog installation position of the jaw part model 11.

The third invention relates to a method for obtaining analog guide manufacturing information.

This method is a method for obtaining information including three-dimensional information of the analog guide required for manufacturing the analog guide.

This method includes a 3D scan image obtaining step, a 3D scan image analyzing step, and an analog guide manufacturing information obtaining step.

The 3D scan image obtaining step is a step for obtaining a 3D scan image 25 of a jaw part 23 of a patient to which a scanning jig 21 is embedded.

The 3D scan image analyzing step is a step for analyzing the 3D scan image 25 of the jaw part 23 obtained in the 3D scan image obtaining step to obtain jaw part information and jig part information.

The analog guide manufacturing information obtaining step is a step for obtaining the analog guide manufacturing information using the jaw part information and the jig part information obtained in the 3D scan image analyzing step.

The fourth invention relates to a method for manufacturing an analog guide.

This method for manufacturing the analog guide includes a step of manufacturing the analog guide 7 using the analog guide manufacturing information obtained based on the above-described method for obtaining the analog guide manufacturing information.

The fifth invention relates to a method for manufacturing an implant 3D replica.

This method includes a 3D scan image obtaining step, a 3D scan image analyzing step, an analog guide manufacturing information obtaining step, an analog guide manufacturing step, a jaw part model manufacturing information obtaining step, a jaw part model manufacturing step, and a dental analog implanting step.

The 3D scan image obtaining step is a step for obtaining the 3D scan image 25 of the jaw part 23 of the patient to which the scanning jig 21 is embedded.

The 3D scan image analyzing step is a step for analyzing the 3D scan image 25 of the jaw part 23 obtained in the 3D scan image obtaining step to obtain the jaw part information and the jig part information.

The analog guide manufacturing information obtaining step is a step for obtaining analog guide 7 manufacturing information using the jaw part information and the jig part information obtained in the 3D scan image analyzing step.

The analog guide manufacturing step is a step for manufacturing the analog guide 7 using the analog guide 7 manufacturing information obtained in the analog guide manufacturing information obtaining step.

The jaw part model manufacturing information obtaining step is a step for obtaining jaw part model manufacturing information based on the jaw part information obtained in the 3D scan image analyzing step.

The jaw part model manufacturing step is a step for manufacturing the jaw part model based on the jaw part model manufacturing information obtained in the jaw part model manufacturing information obtaining step.

The dental analog implanting step is a step for mounting the dental analog 3 on the analog holding jig 13 to implant the dental analog 3 into the jaw part model using the analog guide 7.

### ADVANTAGEOUS EFFECTS OF THE INVENTION

A jig or the like that facilitates installing the dental analog to the jaw part model can be provided.

### BRIEF DESCRIPTION OF THE DRAWINGS

Fig. 1 is a conceptual diagram of an analog holding jig.
Fig. 2 is a conceptual diagram of an analog guide.
Fig. 3 is a conceptual diagram illustrating a state of the analog holding jig being mounted on the analog guide.
Fig. 4 is a conceptual diagram illustrating a state of a dental analog, the analog holding jig, and the analog guide being mounted.
Fig. 5 is a conceptual diagram illustrating an example of a 3D scan image of a jaw part of a patient to which a scanning jig is embedded.
Fig. 6 is a conceptual diagram illustrating a state of the dental analog being mounted on the analog holding jig and the dental analog is implanted into the jaw part model using the analog guide.
Fig. 7 is a drawing illustrating scan data inside an oral cavity.
Fig. 8 is a conceptual diagram illustrating digital scan data.
Fig. 9 is a conceptual diagram illustrating a position of an implant body and a jaw shape.
Fig. 10 is a conceptual diagram illustrating shape data of the analog guide.
Fig. 11 is a photograph substituting for a drawing of the analog guide obtained in a working example.
Fig. 12 is a conceptual diagram illustrating replica data of the jaw part.
Fig. 13 is a conceptual diagram illustrating design data of a jaw model.
Fig. 14 is a photograph substituting for a drawing illustrating the jaw part model.
Fig. 15 is a photograph substituting for a drawing illustrating a gum replica.
Fig. 16 is a photograph substituting for a drawing illustrating a state of fitting the analog guide to the jaw part model.
Fig. 17 is a drawing illustrating a state of simulation of fitting the analog guide to the jaw part model.

### DESCRIPTION OF PREFERRED EMBODIMENTS

The following describes embodiments for executing the present invention using the drawings. The present invention is not limited to the embodiments described below, and includes the ones that are modified as necessary by those skilled in the art within a scope obvious from the following embodiments.

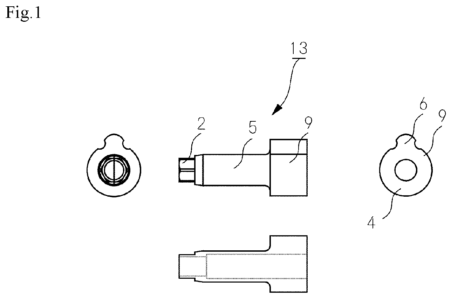

Fig. 1 is a conceptual diagram of an analog holding jig. The analog holding jig is a device (pickup device: PUD) used for holding a dental analog 3 and implanting the dental analog 3 into a jaw part model 11 via an analog guide 7. The dental analog is a jig also referred to as an implant analog (see Japanese Patent No. 6161048).

As illustrated in Fig. 1, an analog holding jig 13 includes a holding portion main body 5 for holding the dental analog 3 and an analog guide connecting portion 9 for coupling to the holding portion main body 5 and connecting with the analog guide 7.

In an example of Fig. 1, the holding portion main body 5 includes a tubular trunk portion and an engaging portion 2 positioned on the upper portion of the trunk portion and having a diameter smaller than that of the trunk portion. The engaging portion 2 is inserted into the dental analog 3 to allow fixing the dental analog 3.

In the example of Fig. 1, the analog guide connecting portion 9 is connected to an end portion opposite to a side of the engaging portion 2 engaging the dental analog 3 in the holding portion main body 5. The analog guide connecting portion 9 includes a disc-shaped hem part 4 and a protrusion portion 6 protruding from the hem part in an outer peripheral direction. The hem part may have a disk shape in multiple stages (a shape having two or more parts on a disk in which the diameter decreases as it approaches the center). The hem part 4 and the protrusion portion 6 of the analog guide connecting portion 9 engage with an analog holding jig connecting portion 17 present in the analog guide 7 having a depression corresponding to them. Then, the analog holding jig 13 is fixed to the analog guide 7.

The analog holding jig 13 (pickup device: PUD) can be manufactured using the publicly known material having a certain amount of strength (for example, plastic). The analog holding jig 13 (PUD) is used for implanting the dental analog into the jaw part model. Therefore, the size of the analog holding jig 13 (PUD) only needs to be appropriately adjusted considering the size of the jaw and the size of the portion of the tooth. The analog holding jig 13 only needs to have a certain amount of strength, and only needs to be appropriately manufactured using the publicly known material (for example, a metal, such as stainless steel, and plastic).

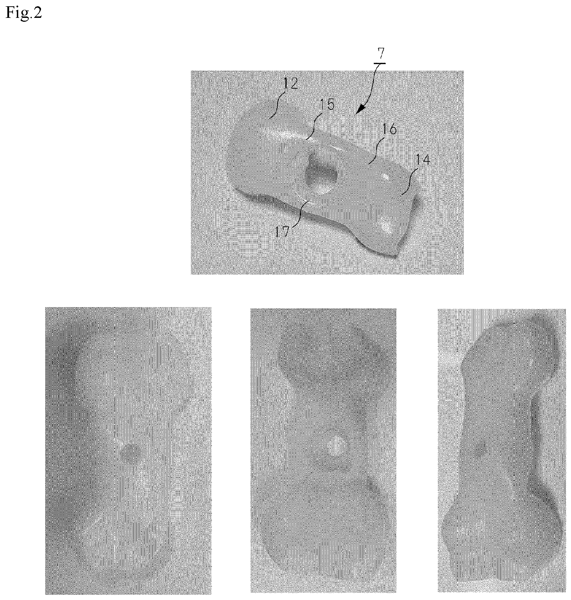

Fig. 2 is a conceptual diagram of the analog guide. The analog guide 7 is a device (pickup guide holder: PUD holder) used for implanting the dental analog 3 into the jaw part model 11. As illustrated in Fig. 2, the analog guide 7 includes an analog guide main body 15 and the analog holding jig connecting portion 17.

The analog guide main body 15 is a portion having a shape to be installable to the jaw part model 11. In addition to the analog holding jig connecting portion 17, the analog guide main body 15 includes crown engaging portions 12, 14 that engage with all or a part of a crown portion adjacent to a portion where the dental analog 3 is inserted and the implant is buried and a coupling portion 16 that couples the crown engaging portions 12, 14 and the analog holding jig connecting portion 17. By engaging the crown engaging portions 12, 14 with the crown portion of the jaw part model 11, the analog guide is fixed to the jaw part model 11. Then, the analog holding jig connecting portion 17 is also fixed to the jaw part model 11, and the analog holding jig 13 and the dental analog 3 are fixed to the jaw part model 11 as well. Thus, the analog guide main body 15 preferably has the shape of reflecting the implant buried portion of a patient as a target (or a jaw model of the patient) and the shape of the tooth adjacent to it.

The analog holding jig connecting portion 17 is a portion disposed in the analog guide main body 15, connected to the analog guide connecting portion 9 of the analog holding jig 13, and for causing the dental analog 3 held to the analog holding jig 13 to face an analog installation position of the jaw part model 11. The analog holding jig connecting portion 17 has a depression corresponding to the hem part 4 and the protrusion portion 6 of the analog guide connecting portion 9 of the analog holding jig 13. The analog guide connecting portion 9 is fitted in the analog holding jig connecting portion 17, and the analog holding jig 13 is fixed to the analog guide 7.

The analog guide 7 (PUD holder) can be manufactured using the publicly known material having a certain amount of strength (example: plastic). The analog guide 7 (PUD holder) may have only one analog holding jig connecting portion 17. Additionally, when a plurality of the implants are disposed, the analog guide 7 (PUD holder) may include the two or more analog holding jig connecting portions 17. The size of the analog guide 7 (PUD holder) only needs to be appropriately adjusted considering the size of the jaw, the size of the portion of the tooth, and the number of analog holding jig connecting portions.

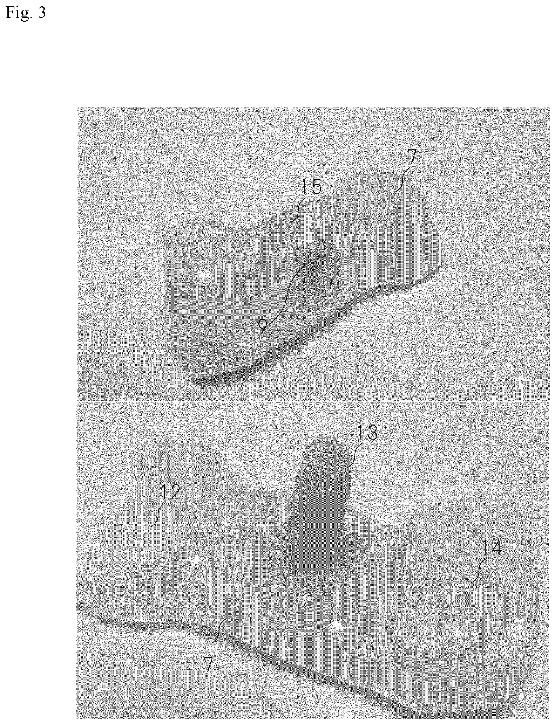

Fig. 3 is a conceptual diagram illustrating a state of the analog holding jig being mounted on the analog guide. As illustrated in Fig. 3, the analog guide connecting portion 9 is fitted to the analog holding jig connecting portion 17, and the analog holding jig 13 is fixed to the analog guide 7.

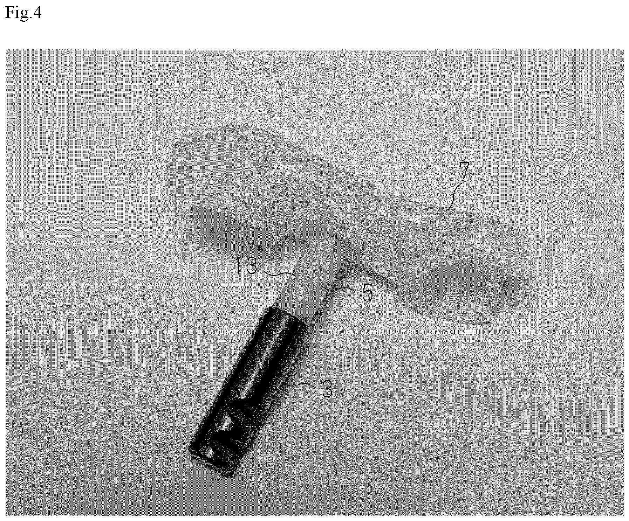

Fig. 4 is a conceptual diagram illustrating a state of the dental analog, the analog holding jig, and the analog guide being mounted.

Next, an example of a method for manufacturing the analog guide will be described. In this example, first, information including three-dimensional information of the analog guide required to manufacture the analog guide is obtained. This method includes a 3D scan image obtaining step, a 3D scan image analyzing step, and an analog guide manufacturing information obtaining step.

The 3D scan image obtaining step is a step for obtaining a 3D scan image 25 of a jaw part 23 of a patient to which a scanning jig 21 is embedded.

The scanning jig 21 is publicly-known, for example, as described in Japanese Patent No. 6208905. An implant body or an intermediate structure of a dental implant is buried in a human or a replica. The scanning jig is mounted on the dental implant. Then, the scanning jig is photographed using, for example, scanner, radiography, and CT. Thus, a system can obtain the 3D scan image 25 of the jaw part 23 of the patient to which the scanning jig 21 is embedded.

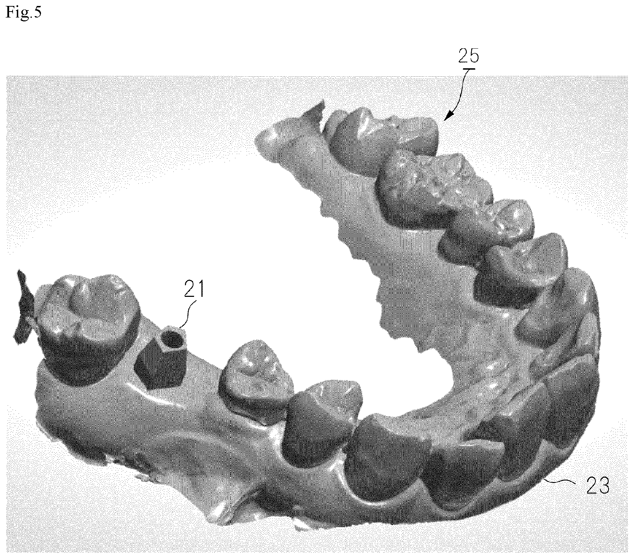

Fig. 5 is a conceptual diagram illustrating an example of the 3D scan image of the jaw part of the patient to which the scanning jig is embedded.

The 3D scan image analyzing step is a step for analyzing the 3D scan image 25 of the jaw part 23 obtained in the 3D scan image obtaining step to obtain jaw part information and jig part information.

In the previous step, the photographed information is transmitted to a computer, and apex parts of a scanned region are obtained from, for example, its contrast. The computer preliminarily stores information on respective side surfaces of a head portion (for example, a shape of a polygonal shape constituting the side surfaces, the adjacent side surface, an area, a position, and an angle). The computer identifies the apex included in the photographed image is which apex of the head part from the obtained apex position information. Then, the area of the polygonal shape formed by the respective apexes is appropriately compared to confirm that analysis of the apexes is correct. The computer obtains the position and the direction of the scanning jig from the information on the positions of the apexes, and obtains the position and the direction of the dental implant coupled to it.

The analog guide manufacturing information obtaining step is a step for obtaining the analog guide manufacturing information using the jaw part information and the jig part information obtained in the 3D scan image analyzing step.

In the previous step, the system obtains the jaw part information and the jig part information. A storage unit appropriately stores the obtained information. The system reads a program from the storage unit, reads the jaw part information and the jig part information from the storage unit, causes a calculation unit to execute arithmetic processing based on a command of the program, and obtains the analog guide manufacturing information. The jaw part information includes information on the shape of the crown portion adjacent to the portion where the implant is buried and the position information. Using the information, the system obtains the whole size of the analog guide 7 (PUD holder) and the shapes of the crown engaging portions 12 and 14. The jig part information includes the position information of the crown portion adjacent to the portion where the implant is buried and information on the direction of burying the implant. Using the information, the system can obtain design information of the analog holding jig connecting portion 17 in the analog guide 7 (PUD holder). Then, the system can obtain information on the coupling portion from the information on the whole size of the analog guide 7 (PUD holder) and the shapes of the crown engaging portions 12 and 14 and the design information of the analog holding jig connecting portion 17. Thus, the system can obtain the analog guide manufacturing information.

Next, the analog guide 7 is manufactured using the analog guide manufacturing information. For example, the system includes a 3D printer. The system causes a 3D printer unit to manufacture the analog guide 7 using the analog guide manufacturing information. Thus, the system can manufacture the analog guide 7. Further, the system may include a cutting machine and may manufacture the analog guide 7 by causing the cutting machine to perform cutting processing using the analog guide manufacturing information.

Next, a method for manufacturing an implant 3D replica (jaw part) will be described.

This method includes a 3D scan image obtaining step, a 3D scan image analyzing step, an analog guide manufacturing information obtaining step, an analog guide manufacturing step, a jaw part model manufacturing information obtaining step, a jaw part model manufacturing step, and a dental analog implanting step. The 3D scan image obtaining step, the 3D scan image analyzing step, the analog guide manufacturing information obtaining step, and the analog guide manufacturing step are as described above.

The jaw part model manufacturing information obtaining step is a step for obtaining jaw part model manufacturing information based on the jaw part information obtained in the 3D scan image analyzing step.

In the previous step, the system obtains the jaw part information. The storage unit appropriately stores the obtained information. The system reads the program from the storage unit, reads the jaw part information from the storage unit, causes the calculation unit to execute arithmetic processing based on a command of the program, and obtains the jaw part model manufacturing information. The jaw part model may be a model of a part of a jaw including an upper jaw and an underjaw, may be a model of only a portion where the implant is buried or only a part of a tooth adjacent to that portion. In addition to the information on the portion where the implant is buried and the shape of the crown portion adjacent to it, the jaw part information includes shape information of the whole jaw. Using the information, the system obtains the size of the whole jaw part model and the shape of the crown. Using the information, the system can obtain the design information of the jaw part model.

The jaw part model manufacturing step is a step for manufacturing the jaw part model based on the jaw part model manufacturing information obtained in the jaw part model manufacturing information obtaining step. Next, the jaw part model is manufactured using the jaw part model manufacturing information. For example, the system includes the 3D printer. The system causes the 3D printer unit to manufacture the jaw part model using the jaw part model manufacturing information. Thus, the system can manufacture the jaw part model.

The dental analog implanting step is a step for mounting the dental analog 3 on the analog holding jig 13 to implant the dental analog 3 into the jaw part model using the analog guide 7.

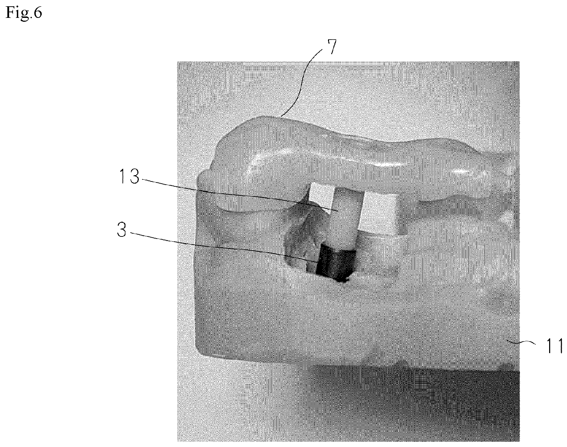

Fig. 6 is a conceptual diagram illustrating a state of the dental analog 3 being mounted on the analog holding jig 13 and the dental analog 3 is implanted into the jaw part model 11 using the analog guide 7.

### WORKING EXAMPLE

The following will specifically describe the present invention using the working example.

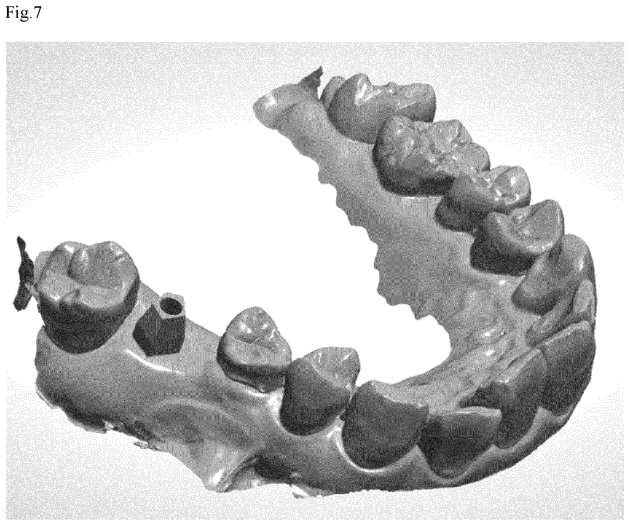

A scanning jig was mounted on an implant body inside an oral cavity to obtain scan data inside the oral cavity including a scanning jig using an intraoral scanner (Fig. 7). Fig. 7 is a drawing illustrating the scan data inside the oral cavity.

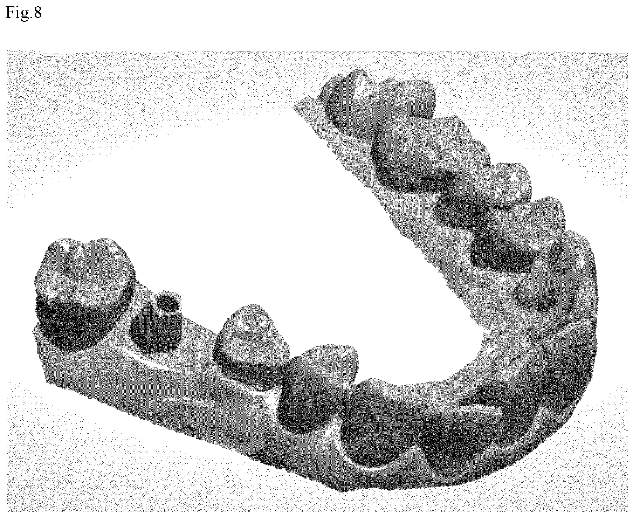

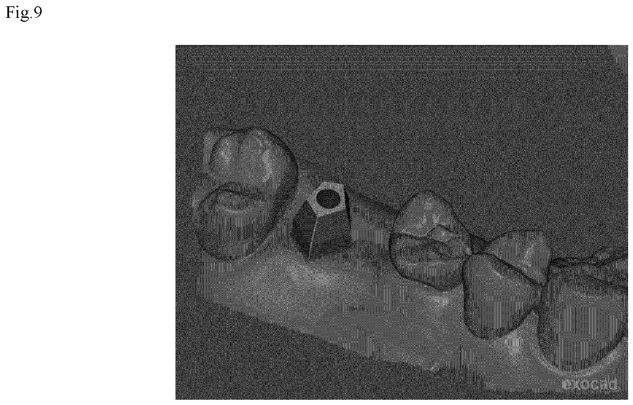

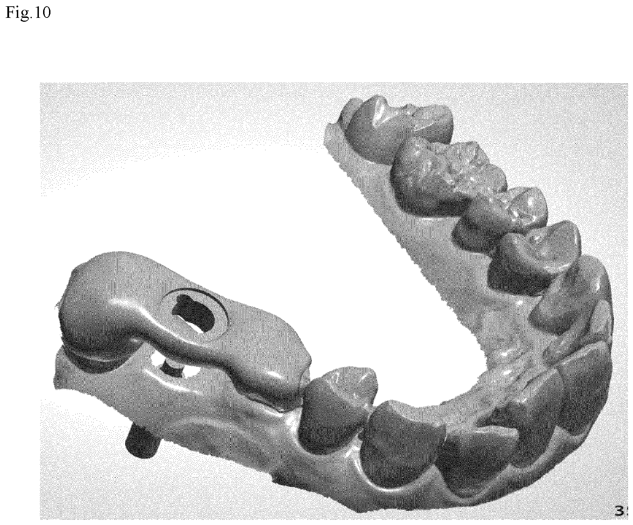

The scan data was converted into data identifying the implant body position (Fig. 8). Fig. 8 is a conceptual diagram illustrating the digital scan data. Shape data of an analog guide was designed (Fig. 10) from the position of the implant body and a jaw shape (Fig. 9) to obtain this data. Fig. 9 is a conceptual diagram illustrating the position of the implant body and the jaw shape. Fig. 10 is a conceptual diagram illustrating shape data of an analog guide.

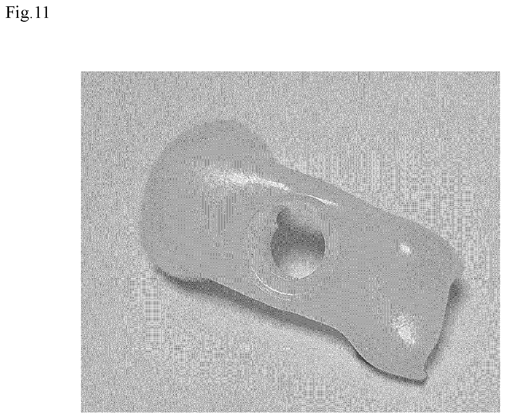

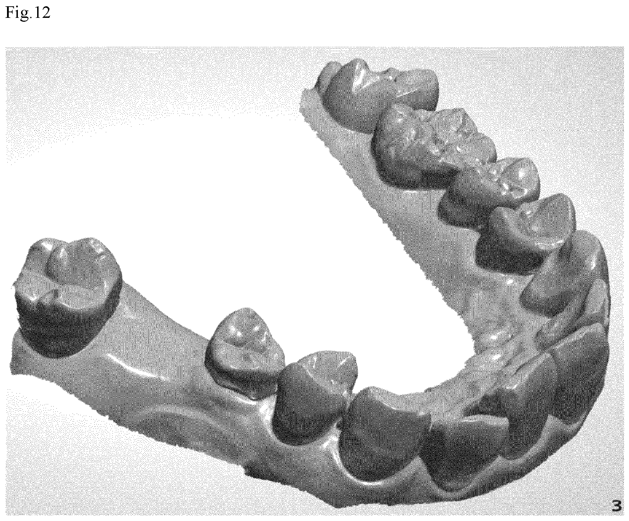

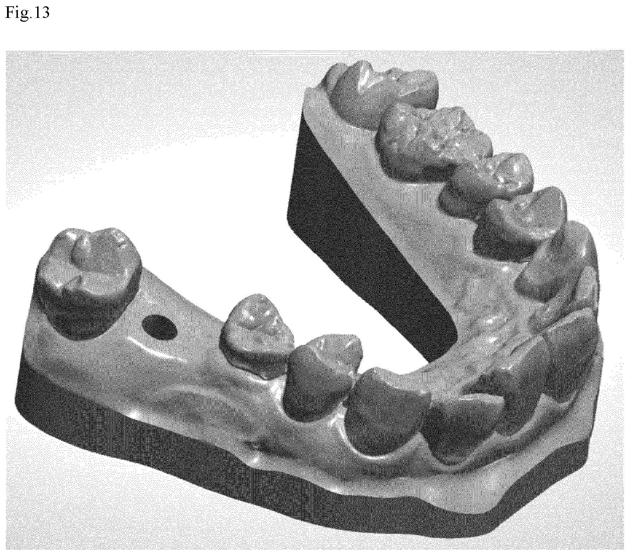

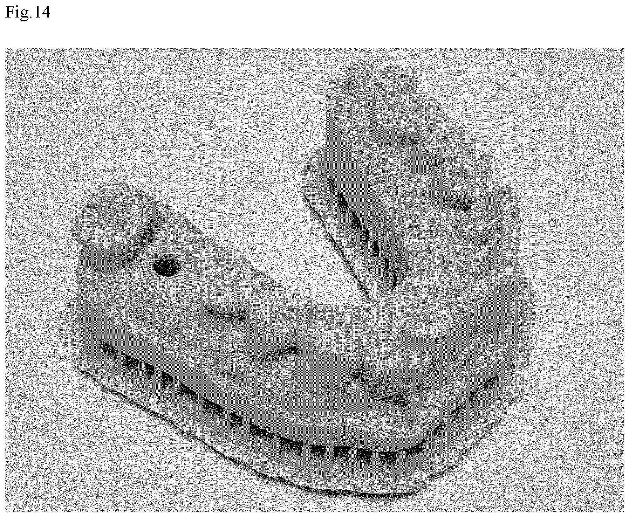

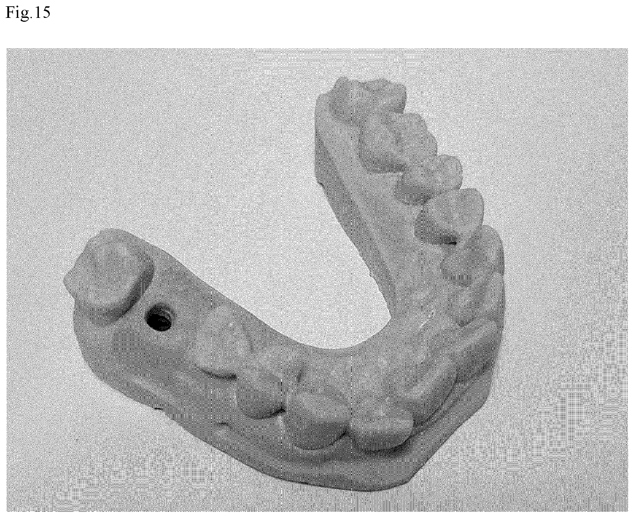

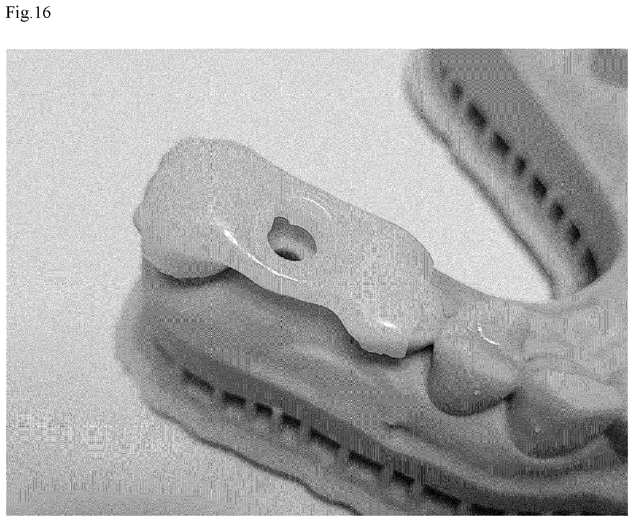

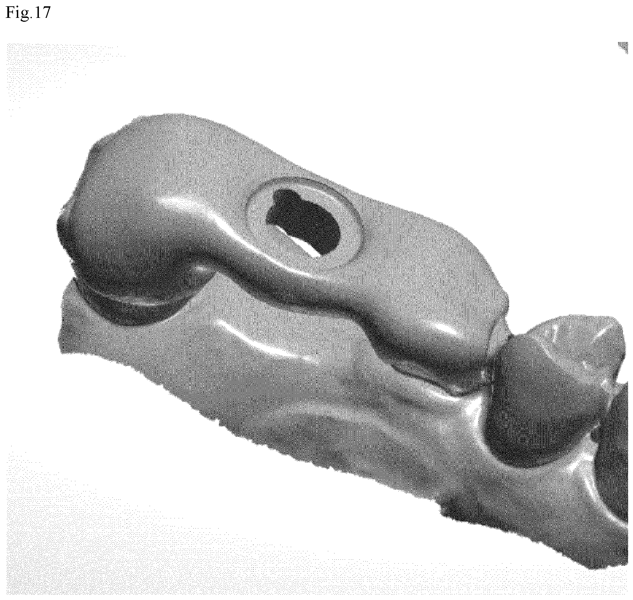

An analog guide was processed from a wax disc (YAMAHACHI DENTAL MFG.,CO., Green T20) using a cutting machine (ROLAND, dwx51) (Fig. 11). Fig. 11 is a photograph substituting for a drawing of the obtained analog guide. From the previous data, replica data of the jaw model (Fig. 12) and replica data formed by cutting the peripheral area of the implant body position were designed (Fig. 13). Fig. 12 is a conceptual diagram illustrating the replica data of the jaw part. Fig. 13 is a conceptual diagram illustrating the design data of the jaw model. The designed replica data was output from the 3D printer (Formlabs, Form3) (Fig. 14). Fig. 14 is a photograph substituting for a drawing illustrating the jaw part model. For improvement in future work efficiency, a gum replica in which the peripheral area of the implant was replaced by a pseudo gingiva replica was produced (Fig. 15). Fig. 15 is a photograph substituting for a drawing illustrating the gum replica. Fitting the analog guide to the 3D printer replica was confirmed from the real objects and scan data after fitting (Fig. 16, Fig. 17). Fig. 16 is a photograph substituting for a drawing illustrating a state of fitting the analog guide to the jaw part model. Fig. 17 is a drawing illustrating a state of simulation of fitting the analog guide to the jaw part model. After the fitting was confirmed, the analog holding jig was mounted on the analog guide, and an implant analog was mounted on the analog holding jig. In the state, the analog guide was mounted on the 3D printer replica, resin (GC, FIXPEED) was cast between the analog and the replica and fixed. Through the operation, a 3D printer implant replica to which the positions obtained from the intraoral scan data were accurately transferred was able to be manufactured.

### INDUSTRIAL APPLICABILITY

This invention can be used in fields of dentistry and medical instruments.

### DESCRIPTION OF REFERENCE SIGNS

- 3: dental analog
- 5: holding portion main body
- 7: analog guide
- 9: analog guide connecting portion
- 11: jaw part model
- 13: analog holding jig
- 15: analog guide main body
- 17: analog holding jig connecting portion
- 21: scanning jig
- 23: jaw part
- 25: 3D scan image

## Claims

1. An analog holding jig (13) comprising:
a holding portion main body (5) for holding a dental analog (3); and
an analog guide connecting portion (9) coupled to the holding portion main body (5) for connecting to an analog guide (7), wherein
the analog holding jig (13) is used for implanting the dental analog (3) into a jaw part model (11) via the analog guide (7).

2. An analog guide (7) used for implanting a dental analog (3) into a jaw part model (11), comprising:
an analog guide main body (15) having a shape to be installable to the jaw part model (11); and
an analog holding jig connecting portion (17) disposed in the analog guide main body (15), the analog holding jig connecting portion (17) being connected to an analog guide connecting portion of an analog holding jig (13) to cause the dental analog (3) held to the analog holding jig (13) to face an analog installation position of the jaw part model (11).

3. A method for obtaining analog guide (7) manufacturing information, comprising:
a 3D scan image obtaining step of obtaining a 3D scan image (25) of a jaw part (23) of a patient to which a scanning jig (21) is embedded;
a 3D scan image analyzing step of analyzing the 3D scan image (25) of the jaw part (23) obtained in the 3D scan image obtaining step to obtain jaw part information and jig part information; and
an analog guide manufacturing information obtaining step of obtaining analog guide (7) manufacturing information using the jaw part information and the jig part information obtained in the 3D scan image analyzing step.

4. A method for manufacturing an analog guide, comprising:
a step of manufacturing the analog guide (7) using the analog guide manufacturing information obtained based on the method for obtaining the analog guide manufacturing information according to claim 3.

5. A method for manufacturing an implant 3D replica, comprising:
a 3D scan image obtaining step of obtaining a 3D scan image (25) of a jaw part (23) of a patient to which a scanning jig (21) is embedded;
a 3D scan image analyzing step of analyzing the 3D scan image (25) of the jaw part (23) obtained in the 3D scan image obtaining step to obtain jaw part information and jig part information;
an analog guide manufacturing information obtaining step of obtaining analog guide (7) manufacturing information using the jaw part information and the jig part information obtained in the 3D scan image analyzing step;
an analog guide manufacturing step of manufacturing the analog guide (7) using the analog guide (7) manufacturing information obtained in the analog guide manufacturing information obtaining step;
a jaw part model manufacturing information obtaining step of obtaining jaw part model manufacturing information based on the jaw part information obtained in the 3D scan image analyzing step;
a jaw part model manufacturing step of manufacturing a jaw part model based on the jaw part model manufacturing information obtained in the jaw part model manufacturing information obtaining step; and
a dental analog implanting step of mounting a dental analog (3) on an analog holding jig (13) to implant the dental analog (3) to the jaw part model using the analog guide (7).
